(19) Europäisches Patentamt
European Patent Office
Office européen des brevets

(11) **EP 3 200 606 B1**

(12) **EUROPÄISCHE PATENTSCHRIFT**

(45) Veröffentlichungstag und Bekanntmachung des Hinweises auf die Patenterteilung:
**31.03.2021 Patentblatt 2021/13**

(21) Anmeldenummer: **15775405.2**

(22) Anmeldetag: **22.09.2015**

(51) Int Cl.:
*A23K 10/00* *(2016.01)*    *A23K 10/40* *(2016.01)*
*A23K 50/00* *(2016.01)*    *A61K 31/20* *(2006.01)*
*A23K 40/30* *(2016.01)*    *A23K 10/12* *(2016.01)*
*A23K 10/16* *(2016.01)*    *A23K 20/158* *(2016.01)*
*A23K 20/163* *(2016.01)*    *A23K 40/25* *(2016.01)*
*A23K 50/80* *(2016.01)*

(86) Internationale Anmeldenummer:
**PCT/EP2015/071707**

(87) Internationale Veröffentlichungsnummer:
**WO 2016/050559 (07.04.2016 Gazette 2016/14)**

(54) **VERFAHREN ZUR HERSTELLUNG EINES PUFAS ENTHALTENDEN FUTTERMITTELS DURCH EXTRUSION EINER PUFAS ENTHALTENDEN BIOMASSE DES TYPS LABYRINTHULOMYCETES**

METHOD FOR PRODUCING FEED CONTAINING PUFAS BY EXTRUSION OF A BIOMASS CONTAINING PUFAS OF THE LABYRINTHULOMYCETES TYPE

PROCEDE DE PRODUCTION D'ALIMENTS POUR ANIMAUX CONTENANT DES PUFAS PAR EXTRUSION D'UNE BIOMASSE CONTENANT DES PUFAS DU TYPE LABYRINTHULOMYCÈTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2014 EP 14187479**

(43) Veröffentlichungstag der Anmeldung:
**09.08.2017 Patentblatt 2017/32**

(73) Patentinhaber: Evonik Operations GmbH
**45128 Essen (DE)**

(72) Erfinder:
• **RABE, Christian**
**63762 Großostheim (DE)**
• **SILVA, Amelia Claudia**
**63457 Hanau (DE)**
• **EILS, Stefan**
**63584 Gründau (DE)**
• **PRIEFERT, Horst**
**48346 Ostbevern (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
WO-A1-01/54510    WO-A1-94/08467
WO-A1-2011/006261    WO-A1-2014/122092
GB-A- 2 324 701

• **Rajagopalan Prabu ET AL: "Effect of sodium sulphate salinity for production of docosahexaenoic acid (DHA) by Thraustochytrids aureum RAK-21", Asian Biomedicine, Bd. 6, Nr. 5 5. Oktober 2012 (2012-10-05), Seiten 693-701, XP055163274, DOI: 10.5372/1905-7415.0605.109 Gefunden im Internet: URL:http://abm.digitaljournals.org/index.php/abm/article/viewFile/1410/629 [gefunden am 2015-01-20]**

**Beschreibung**

Die vorliegende Erfindung betrifft ein Verfahren zur Herstellung eines polyungesättigte Fettsäuren (PUFAs) enthaltenden Futtermittels durch Extrusion einer PUFAs enthaltenden Biomasse.

Verfahren zur Herstellung von PUFAs (polyungesättigte Fettsäuren) enthaltender Biomasse sind im Stand der Technik bereits beschrieben. Die WO2014/122092 beschreibt Extrudate, die PUFAs enthaltende Thraustochytriales Biomasse enthalten. Der Sulfatgehalt der Biomasse liegt unter 25g/kg. Die WO94/08467 beschreibt Kultivierung von Schizochytrium ATCC No. 20888 unter verschiedenen $Na_2SO_4$ Gehalten im Medium. Die Biomasse kann zu einem Extrudat verarbeitet werden. Die Weiterverarbeitung entsprechender Biomasse gemeinsam mit anderen Futtermittel-Inhaltsstoffen kann beispielsweise durch Extrusion erfolgen. Die PUFAs enthaltende Biomasse wird hierbei als alternative Quelle für PUFAs verwendet, da aufgrund von Überfischung Fischöl, das klassischer Weise als Quelle für PUFAs verwendet wird, nicht mehr in ausreichender Menge zur Verfügung steht.

Aufgabe der vorliegenden Erfindung war es, ein PUFAs, insbesondere omega-3-Fettsäuren, enthaltendes Futtermittel zur Verfügung zu stellen, das eine möglichst hohe Ölbeladungskapazität aufweist. Denn nachträglich mit Öl beschichtete Futtermittel haben sich als besonders geeignete Futtermittel, insbesondere in der Aquakultur, herausgestellt.

Es wurde zuvor festgestellt, dass bei Verwendung von Fischöl für die Herstellung des Futtermittels bei Durchführung der Extrusion ein sehr hoher Energieeintrag erforderlich ist, um ein Produkt mit einer gewünschten Ölbeladungskapazität zu erhalten. Bei der Verwendung von Fischöl als Quelle für omega-3-Fettsäuren erfolgt zunächst Extrusion der übrigen Futtermittelkomponenten und das so erhaltene Extrudat wird anschließend mit Fischöl sowie gegebenenfalls weiteren Öl-Komponenten beladen.

Überraschenderweise wurde erfindungsgemäß nun festgestellt, dass bei Verwendung einer PUFAs, insbesondere omega-3-Fettsäuren, enthaltenden Biomasse gemäß Anspruch 1 anstelle von Fischöl ein sehr viel niedriger Energieeintrag ausreicht, um ein Extrudat mit gewünschter Ölbeladungskapazität zu erhalten. Ein Energieeintrag von maximal 28 Wh/kg hat sich hierbei als ausreichend zum Erhalt der gewünschten Produkteigenschaften herausgestellt, wobei ein Energieeintrag von 12-28 Wh/kg vorteilhaft ist.

Ein besonders niedriger Energieeintrag von maximal 22 Wh/kg, insbesondere 18-22 Wh/kg, ist insbesondere dann ausreichend, wenn zur Herstellung des Futtermittels eine PUFAs enthaltende Biomasse gemäß Anspruch 1 verwendet wird, die durch Anzucht bei einer Sulfat-Konzentration erhalten wurde, so dass in der resultierenden Biomasse, in Bezug auf die Trockenmasse, ein Sulfat-Gehalt von 25 bis 60 g/kg, insbesondere 25 bis 50, 25 bis 40 oder 25 bis 35 g/kg, vorliegt.

Ein erster Gegenstand der vorliegenden Erfindung ist daher ein Verfahren zur Herstellung eines PUFAs enthaltenden Futtermittels, dadurch gekennzeichnet, dass eine PUFAs enthaltende Biomasse des Taxons Labyrinthulomycetes, die einen Sulfat-Gehalt, bezogen auf die Trockenmasse, von 25 bis 60 g/kg aufweist, mit weiteren Komponenten des Futtermittels bei einem Energieeintrag von 12 - 28 Wh/kg extrudiert wird, wobei unter Trockenmasse ein Produkt mit einer Restfeuchte von unter 5 Gew.-% zu verstehen ist und und wobei der Sulfat-Gehalt der Biomasse über den Gehalt an Schwefel in der Biomasse und dieser wiederum nach DIN EN ISO 11885 ermittelt wird. Die Extrusion erfolgt hierbei vorzugsweise bei einem Energieeintrag von 14 - 26, insbesondere 16-24, besonders bevorzugt 18-22 Wh/g. Der Sulfat-Gehalt der Biomasse beträgt, bezogen auf die Trockenmasse, vorzugsweise 25 bis 50, insbesondere 25 bis 40, besonders bevorzugt 25 bis 35 g/kg, wobei unter Trockenmasse ein Produkt mit einer Restfeuchte von unter 5 Gew.-% zu verstehen ist und und wobei der Sulfat-Gehalt der Biomasse über den Gehalt an Schwefel in der Biomasse und dieser wiederum nach DIN EN ISO 11885 ermittelt wird.

Im Extrusionsverfahren wird hierbei vorzugsweise ein Schnecken- oder Doppelschneckenextruder eingesetzt. Das Extrusionsverfahren wird vorzugsweise bei einer Temperatur von 80 - 220° C, insbesondere 80 - 130 °C, einem Druck von 10 - 40 bar, und einer Wellenumlaufgeschwindigkeit von 100 - 1000 rpm, insbesondere 300 - 700 rpm, durchgeführt. Die Verweilzeit des eingebrachten Gemisches beträgt vorzugsweise 5 - 30 Sekunden, insbesondere 10 - 20 Sekunden.

Das erzeugte Extrudat hat vorzugsweise einen Durchmesser von 1 bis 14 mm, vorzugsweise 2 bis 12 mm, insbesondere 2 bis 6 mm, und weist vorzugsweise auch eine Länge von 1 bis 14 mm, vorzugsweise 2 bis 12 mm, insbesondere 2 bis 6 mm, auf. Die Länge des Extrudats wird durch Einsatz eines Schneidwerkzeugs während der Extrusion eingestellt. Die Länge des Extrudats wird vorzugsweise so gewählt, dass es dem Durchmesser des Extrudats in etwa entspricht. Der Durchmesser des Extrudats wird durch Wahl des Siebdurchmessers festgelegt.

Die Ölbeladungskapazität steht in unmittelbarem Zusammenhang mit der Expansion des Extrudats während des Extrusionsvorgangs. Je stärker die Expansion bei der Extrusion, desto größer die Öl-Beladungskapazität des erhaltenen Extrudats.

Die Öl-Beladungskapazität eines erfindungsgemäßen Extrudats beträgt vorzugsweise mindestens 0,25 g Öl pro g Extrudat, besonders bevorzugt mindestens 0,3 g Öl pro g Extrudat, insbesondere mindestens 0,35 g Öl pro g Extrudat.

Weiterer Gegentand der vorliegenden Erfindung ist daher ebenso ein PUFAs enthaltendesFuttermittelextrudat, das eine PUFAs enthaltende Biomasse des Taxons Labyrinthulomycetes enthält, dadurch gekennzeichnet, dass die Biomasse einen Sulfat-Gehalt, bezogen auf die Trockenmasse, von 25 bis 60 g/kg aufweist und dass das Extrudat eine Ölbeladungskapazität von mindestens 0,25 g Öl pro g Extrudat, insbesondere mindestens 0,275 oder 0,30 g Öl pro g Extrudat, vorzugsweise mindestens 0,325 oder 0,35 g Öl pro g Extrudat, aufweist, wobei unter Trockenmasse ein Produkt mit einer Restfeuchte von unter 5 Gew.-% zu verstehen ist und und wobei der Sulfat-Gehalt der Biomasse über den Gehalt an Schwefel in der Biomasse und dieser wiederum nach DIN EN ISO 11885 ermittelt wird.

Auf diese Weise lassen sich durch Ausschöpfung der Öl-Beladungskapazität Extrudate mit einem Gesamt-Fettgehalt von mindestens 25 Gew.-% oder mindestens 27,5 Gew.-%, insbesondere mindestens 30 Gew.-% oder mindestens 32,5 Gew.-%, vor allem auch solche mit einem Gesamt-Fettgehalt von über 35 Gew.-% realisieren.

Das erfindungsgemäße Extrudat weist vorzugsweise eine Schüttdichte von 400 - 500 g/l auf. Das Extrusionsverfahren kann gegebenenfalls einen Kompaktierungs- und/oder einen Kompressionsschritt umfassen.

Vor der Durchführung des Extrusionsverfahrens werden die Komponenten vorzugsweise innig miteinander vermischt. Dies geschieht vorzugsweise in einer Trommel, die mit Schaufeln ausgestattet ist. Bei diesem Mischungsschritt erfolgt in einer bevorzugten Ausführungsform eine Wasserdampfinjektion, insbesondere um die Quellung der vorzugsweise enthaltenen Stärke zu bewirken. Die Wasserdampfinjektion wird hierbei vorzugsweise bei einem Druck von 1 bis 5 bar durchgeführt, besonders bevorzugt bei einem Druck von 2 bis 4 bar.

Die weiteren Nahrungs- oder Futtermittelinhaltsstoffe werden vor dem Vermischen mit der Algen-Biomasse - soweit erforderlich - vorzugsweise zerkleinert, um sicherzustellen, dass bei dem Mischungsschritt ein homogenes Gemisch erhalten wird. Das Zerkleinern der weiteren Nahrungs- oder Futtermittelinhaltsstoffe kann beispielsweise unter Verwendung einer Hammer-Mühle erfolgen.

In einer erfindungsgemäß bevorzugten Ausführungsform schließt sich an das Extrusionsverfahren die Beladung des erhaltenen Extrudats mit Öl an. Hierzu wird das Extrudat vorzugsweise zunächst auf einen Feuchtigkeitsgehalt von maximal 5 Gew.-% getrocknet. Die Beladung des Extrusionsprodukts mit Öl kann erfindungsgemäß beispielsweise durch Einlegen des Extrudats in Öl oder Besprühen des Extrudats mit Öl erfolgen, sie erfolgt erfindungsgemäß jedoch vorzugsweise durch Vakuumbeschichtung.

Die Herstellung erfindungsgemäß einsetzbarer PUFAs enthaltender Biomassen ist im Stand der Technik ausführlich beschrieben. Bei den verwendeten Zellen handelt es sich um Zellen des Taxons Labyrinthulomycetes, die bereits natürlicherweise PUFAs (polyungesättigte Fettsäuren) produzieren. Die Produktion kann hierbei autotroph, mixotroph oder heterotroph erfolgen.

Bei der Biomasse des Taxons Labyrinthulomycetes (Labyrinthulea, Netzschleimpilze, Schleimnetze) handelt es sich vorzugsweise um eine solche der Familie der Thraustochytriaceae. Zu der Familie der Thraustochytriaceae gehören die Gattungen Althomia, Aplanochytrium, Elnia, Japonochytrium, Schizochytrium, Thraustochytrium, Aurantiochytrium, Oblongichytrium und Ulkenia. Besonders bevorzugt handelt es sich bei der Biomasse um eine der Gattungen Thraustochytrium, Schizochytrium, Aurantiochytrium oder Oblongichytrium, vor allem um eine der Gattung Aurantiochytrium.

Innerhalb der Gattung Aurantiochytrium ist erfindungsgemäß die Spezies Aurantiochytrium limacinum (früher auch Schizochytrium limacinum genannt) bevorzugt. Ganz besonders bevorzugt wird erfindungsgemäß der Stamm Aurantiochytrium limacinum SR21 eingesetzt.

Bei der polyungesättigten Fettsäure (PUFA) handelt es sich erfindungsgemäß vorzugsweise um eine hochungesättigte Fettsäure (HUFA).

Die in der Biomasse enthaltenen Zellen zeichnen sich vorzugsweise dadurch aus, dass sie einen PUFA-Gehalt, von mindestens 20 Gew.-%, vorzugsweise mindestens 30 Gew.-%, insbesondere mindestens 35 Gew.-%, jeweils bezogen auf die Zelltrockenmasse, aufweisen.

In einer bevorzugten Ausführungsform liegt hierbei ein Großteil der Lipide in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen Lipide in Form von Triglyceriden vorliegen.

Unter polyungesättigten Fettsäuren (PUFAs) werden erfindungsgemäß Fettsäuren verstanden, die mindestens zwei, insbesondere mindestens drei, C-C-Doppelbindungen aufweisen. Unter den PUFAs sind erfindungsgemäß hochungesättigte Fettsäuren (HUFAs) bevorzugt. Unter HUFAs werden erfindungsgemäß Fettsäuren verstanden, die mindestens vier C-C-Doppelbindungen aufweisen.

Die PUFAs können in der Zelle in freier Form oder in gebundener Form vorliegen. Beispiele für das Vorliegen in gebundener Form sind Phospholipide und Ester der PUFAs, insbesondere Monoacyl-, Diacyl- und Triacylglyceride. In einer bevorzugten Ausführungsform liegt ein Großteil der PUFAs in Form von Triglyceriden vor, wobei vorzugsweise mindestens 50 Gew.-%, insbesondere mindestens 75 Gew.-% und in einer besonders bevorzugten Ausführungsform mindestens 90 Gew.-% der in der Zelle enthaltenen PUFAs in Form von Triglyceriden vorliegen.

Bevorzugte PUFAs stellen omega-3-Fettsäuren und omega-6-Fettsäuren dar, wobei omega-3-Fettsäuren besonders bevorzugt sind. Bevorzugte omega-3-Fettsäuren stellen hierbei die Eicosapentaensäure (EPA, 20:5ω-3), insbesondere die (5Z,8Z,11Z,14Z,17Z)-Eicosa-5,8,11,14,17-pentaensäure, und die Docosahexaensäure (DHA, 22:6ω-3), insbesondere die (4Z,7Z,10Z,13Z,16Z,19Z)-Docosa-4,7,10,13,16,19-hexaensäure, dar, wobei die Docosahexaensäure besonders bevorzugt ist.

Die PUFAs enthaltende Algen-Biomasse macht vorzugsweise 2 bis 24 Gew.-%, insbesondere 4 bis 22 Gew.-%, vorzugsweise 9 bis 20 Gew.-%, vor allem 11 bis 18 Gew.-%, des Extrudats bzw. der zur Herstellung des Extrudats eingesetzten Zusammensetzung aus.

Die anderen Nahrungs- oder Futtermittelinhaltsstoffe sind vorzugsweise ausgewählt aus proteinhaltigen, kohlenhydrathaltigen, nukleinsäurehaltigen und lipidlöslichen Komponenten sowie gegebenenfalls weiteren fetthaltigen Komponenten und weiterhin aus anderen Zusatzstoffen wie Mineralien, Vitaminen, Pigmente und Aminosäuren. Daneben können neben Nährsubstanzen auch strukturgebende Substanzen enthalten sein, um etwa die Textur oder das Erscheinungsbild des Futtermittels zu verbessern. Weiterhin können beispielsweise auch Bindemittel eingesetzt werden, um die Konsistenz des Futtermittels zu beeinflussen. Eine bevorzugt eingesetzte Komponente, die sowohl eine Nährsubstanz als auch eine strukturgebende Substanz darstellt, ist Stärke.

In einem erfindungsgemäß besonders bevorzugten Extrusionsverfahren wird eine Zusammensetzung eingesetzt, die folgende Eigenschaften aufweist:

a) einen Gesamt-Proteingehalt von 33 bis 67 Gew.-%, vorzugsweise 39 bis 61 Gew.-%, insbesondere 44 bis 55 Gew.-%,

b) einen Gesamt-Fettgehalt von 5 bis 25 Gew.-%, vorzugsweise 8 bis 22 Gew.-%, insbesondere 10 bis 20 Gew.-%, vor allem 12 bis 18 Gew.-%;

c) einen Gesamt-Stärkegehalt von maximal 25 Gew.-%, insbesondere maximal 20 Gew.-%, vorzugsweise 6 bis 17 Gew.-%, besonders bevorzugt 8 bis 14 Gew.-%;

d) einen Gehalt an Labyrinthulomycetes-Biomasse insbesondere Thraustochytriaceae-Biomasse, von 2 bis 24 Gew.-%, vorzugsweise 4 bis 22 Gew.-%, insbesondere 9 bis 20 Gew.-%, vor allem 11 bis 18 Gew.-%;

e) vorzugsweise einen Gehalt an polyungesättigten Fettsäuren (PUFAs) von 0,8 bis 8 Gew.-%, vorzugsweise 1,2 bis 6 Gew.-%, insbesondere 1,4 bis 5 Gew.-%, vor allem 1,5 bis 4 Gew.-%;

f) vorzugsweise einen Gehalt an omega-3-Fettsäuren von 0,8 bis 8 Gew.-%, vorzugsweise 1,2 bis 6 Gew.-%, insbesondere 1,4 bis 5 Gew.-%, vor allem 1,5 bis 4 Gew.-%;

g) vorzugsweise Gehalt an DHA von 0,1 bis 4,0 Gew.-%, vorzugsweise 0,25 bis 3,0 Gew.-%, insbesondere 0,5 bis 2,8 Gew.-%, vor allem 0,8 bis 2,5 Gew.-%, insbesondere 1,0 bis 2,0 Gew.-%.

Das erhaltene Extrudat wird anschließend in einer bevorzugten Ausführungsform mit Öl, insbesondere Pflanzenöl, vorzugsweise in einer Menge von 3 bis 18 Gew.-%, insbesondere 5 bis 15 Gew.-%, besonders bevorzugt 7 bis 13 Gew.-%, in Bezug auf das finale Produkt, beschichtet.

Es wird so entsprechend ein ölbeschichtetes Extrudat erhalten, das vorzugsweise folgende Eigenschaften aufweist:

a) einen Gesamt-Proteingehalt von 30 bis 60 Gew.-%, vorzugsweise 35 bis 55 Gew.-%, insbesondere 40 bis 50 Gew.-%;

b) einen Gesamt-Fettgehalt von 15 bis 35 Gew.-%, vorzugsweise 18 bis 32 Gew.-%, insbesondere 20 bis 30 Gew.-%, vor allem 22 bis 28 Gew.-%;

c) einen Gesamt-Stärkegehalt von maximal 25 Gew.-%, insbesondere maximal 20 Gew.-%, vorzugsweise 5 bis 15 Gew.-%, besonders bevorzugt 7 bis 13 Gew.-%;

d) einen Gehalt an Labyrinthulea-Biomasse, insbesondere Thraustochytriaceae-Biomasse, von 2 bis 22 Gew.-%, vorzugsweise 4 bis 20 Gew.-%, insbesondere 8 bis 18 Gew.-%, vor allem 10 bis 16 Gew.-%;

e) einen Gehalt an polyungesättigten Fettsäuren (PUFAs) von 2 bis 12 Gew.-%, vorzugsweise 3 bis 10 Gew.-%, insbesondere 4 bis 9 Gew.-%, vor allem 5 bis 8 Gew.-%;

f) einen Gehalt an omega-3-Fettsäuren von 1 bis 6 Gew.-%, vorzugsweise 1,5 bis 5 Gew.-%, insbesondere 2 bis 4,5 Gew.-%, vor allem 2,5 bis 4 Gew.-%;

g) einen Gehalt an DHA von 0,5 bis 3 Gew.-%, vorzugsweise 0,8 bis 2,5 Gew.-%, insbesondere 1 bis 2,5 Gew.-%, vor allem 1,2 bis 2,2 Gew.-%, insbesondere 1,2 bis 2,0 Gew.-%.

Vor allem weist ein erfindungsgemäß erhaltenes ölbeschichtetes Extrudat folgende Eigenschaften auf:

a) einen Gesamt-Protein-Gehalt von 30 bis 60 Gew.-%, vorzugsweise 35 bis 55 Gew.-%, insbesondere 40 bis 50 Gew.-%;

b) einen Gesamt-Fett-Gehalt von 15 bis 35 Gew.-%, vorzugsweise 18 bis 32 Gew.-%, insbesondere 20 bis 30 Gew.-%, vor allem 22 bis 28 Gew.-%;

c) einen Gesamt-Stärkegehalt von maximal 25 Gew.-%, insbesondere maximal 20 Gew.-%, vorzugsweise 5 bis 15 Gew.-%, besonders bevorzugt 7 bis 13 Gew.-%;

d) einen Gehalt an Aurantiochytrien-Biomasse, insbesondere Aurantiochytrium limacinum-Biomasse, vor allem Aurantiochytrium limacinum SR21-Biomasse, von 2 bis 22 Gew.-%, vorzugsweise 4 bis 20 Gew.-%, insbesondere 8 bis 18 Gew.-%, vor allem 10 bis 16 Gew.-%;

e) einen Gehalt an polyungesättigten Fettsäuren (PUFAs) von 2 bis 12 Gew.-%, vorzugsweise 3 bis 10 Gew.-%, insbesondere 4 bis 9 Gew.-%, vor allem 5 bis 8 Gew.-%;

f) einen Gehalt an omega-3-Fettsäuren von 1 bis 6 Gew.-%, vorzugsweise 1,5 bis 5 Gew.-%, insbesondere 2 bis 4,5 Gew.-%, vor allem 2,5 bis 4 Gew.-%;

g) einen Gehalt an DHA von 0,5 bis 3 Gew.-%, vorzugsweise 0,8 bis 2,5 Gew.-%, insbesondere 1 bis 2,5 Gew.-%, vor allem 1,2 bis 2,2 Gew.-%, insbesondere 1,2 bis 2,0 Gew.-%.

Als fetthaltige Komponente können erfindungsgemäß neben der erfindungsgemäß einzusetzenden Biomasse Fette, insbesondere Öle, tierischen als auch solche pflanzlichen Ursprungs eingesetzt werden. Als fetthaltige Komponente kommen erfindungsgemäß insbesondere Pflanzenöle in Betracht, beispielsweise Sojabohnenöl, Rapssamenöl, Sonnenblumenkernöl, Flachssamenöl oder Palmöl sowie Mischungen davon. Daneben kann gegebenenfalls auch Fischöl als fetthaltige Komponente in geringen Mengen eingesetzt werden.

Vorzugsweise enthält ein erfindungsgemäßes ölbeschichtetes Extrudat pflanzliche Öle in einer Menge von 3 bis 18 Gew.-%, insbesondere 5 bis 15 Gew.-%, vor allem 7 bis 13 Gew.-%. Wie zuvor beschrieben wird das pflanzliche Öl hierbei vorzugsweise nachträglich auf das Extrudat aufgetragen, insbesondere durch Vakuumbeschichtung.

Als proteinhaltige Komponente können erfindungsgemäß beispielsweise Sojaprotein, Erbsenprotein, Weizengluten oder Maisgluten sowie Mischungen davon eingesetzt werden.

Als proteinhaltige Komponente, die zusätzlich Fette enthält, können beispielsweise eingesetzt werden Fischmehl, Krillmehl, Muschelmehl, Kalmarmehl oder Schrimpsschalen. Diese werden im Folgenden unter dem Begriff "marines Mehl" zusammengefasst. In einer bevorzugten Ausführungsform umfasst ein erfindungsgemäßes Futtermittel marines Mehl, vorzugsweise Fischmehl, in einer Menge von 3 bis 18 Gew.-%, insbesondere 5 bis 15 Gew.-%, vor allem 7 bis 13 Gew.-%.

Als kohlenhydrathaltige Komponente können beispielsweise Weizenmehl, Sonnenblumenmehl oder Sojamehl sowie Mischungen davon eingesetzt werden.

Neben der hohen Öl-Beladungskapazität zeichnet sich ein erfindungsgemäßes Extrudat vorzugsweise auch dadurch aus, dass es eine sehr hohe Abriebfestigkeit aufweist. Diese beträgt vorzugsweise mindestens 91 %, insbesondere mindestens 92 oder 93 %, besonders bevorzugt mindestens 94 %.

Die Bestimmung der Abriebfestigkeit erfolgte erfindungsgemäß auf folgende Weise: Das getrocknete Extrudat (mit 4 mm Durchmesser und 4 mm Länge) wurde einer mechanischen Belastung unter Verwendung des Holmen Pellet-Tester NHP100 (Borregaard Lignotech, Hull, UK) ausgesetzt. Vor Durchführung des Tests wurden die Proben gesiebt, um möglicherweise anhaftende Feinteilchen zu entfernen. Die aufbereiteten Proben (100 g) wurden anschließend in den Pellet-Tester eingeführt unter Verwendung eines 2,5 mm-Filtersiebs. Die Pellets wurden anschließend mit hoher Luftgeschwindigkeit (ca. 70 mbar) für 30 Sekunden durch ein Röhrchen mit rechtwinkligen Rohrbogen befördert. Die Versuchsparameter sind gerätebedingt vorgegeben. Anschließend wurde der Abrieb durch Wiegen bestimmt. Die Abriebfestigkeit wurde als PDI (Pellet Durability Index) angegeben, definiert als die Menge an in dem Filtersieb nach Durchführung des Tests verbliebener Probe in Prozent. Der Test wurde jeweils mit drei Proben durchgeführt und anschließend der Mittelwert gebildet.

Das ölbeladene Extrudat zeichnet sich vorzugsweise dadurch aus, dass es eine sehr hohe Wasserstabilität aufweist. Diese beträgt vorzugsweise mindestens 96 %, insbesondere mindestens 97 %, besonders bevorzugt mindestens 98 %.

Die Wasserstabilität wurde mit den ölbeladenen Proben durchgeführt. Die Methode wurde im Wesentlichen ausgeführt wie durch Baeverfjord et al. (2006; Aquaculture 261, 1335-1345) beschrieben, mit geringen Modifikationen. 10 g-Proben des Extrudats (mit einer Länge und einem Durchmesser von jeweils 4 mm) wurden in metallische Infusions-Körbchen (Inox, Deutschland) mit einem Durchmesser von 6,5 mm und einer Maschenweite von 0,3 mm gegeben. Die Infusions-Körbchen wurden anschließend in eine Plastik-Wanne mit Wasser gegeben, so dass die Proben vollständig mit Wasser bedeckt waren. Die Wanne wurde anschließend für 30 Minuten einer Schüttelagitation von 30 Schütteleinheiten pro Minute unter Verwendung des Multiorbital-Schüttlers PSU-20l (Biosan, Lettland) ausgesetzt. Danach wurden die Proben vorsichtig mit Löschpapier getrocknet und anschließend gewogen bevor und nachdem sie einer Ofen-Trocknung bei einer Temperatur von 105 °C für 24 Stunden unterzogen worden waren. Die Wasserstabilität wurde berechnet als die Differenz des Trockengewichts der Probe vor und nach der Inkubation in Wasser und in Prozent des Trockengewichts der eingesetzten Probe vor der Inkubation mit Wasser angegeben.

Bei dem Nahrungs- oder Futtermittel handelt es sich vorzugsweise um ein Mittel zum Einsatz in der Aquakultur oder um ein Nahrungs- oder Futtermittel zum Einsatz in der Geflügelzucht, Schweinezucht oder Rinderzucht. Bei dem Futtermittel kann es sich auch um ein Futtermittel handeln, das eingesetzt wird, um Kleinlebewesen zu züchten, die in der Aquakultur als Futtermittel eingesetzt werden können. Bei den Kleinlebewesen kann es sich beispielsweise um Nematoden, Crustaceen oder Rotiferen handeln. Das Futtermittel liegt vorzugsweise flockenförmig, kugelförmig oder tablettenförmig vor. Ein durch Extrusion erhältliches Futtermittel hat vorzugsweise einen Feuchtigkeitsgehalt von weniger als 5 Gew.-%, besonders bevorzugt von 0,2 bis 4 Gew.-%.

Das Futtermittel zum Einsatz in der Aquakultur wird vorzugsweise verwendet um Flossenfische und Crustaceen zu züchten, die vorzugsweise der Ernährung von Menschen dienen. Hierzu zählen insbesondere Karpfen, Tilapia, Welse, Thunfisch, Lachs, Forellen, Baramundi, Brassen, Barsche, Kabeljau, Schrimps, Hummer, Krabben, Garnelen und Krebse. Besonders bevorzugt handelt es sich um ein Futtermittel für die Lachs-Zucht. Bevorzugte Lachsarten stellen hierbei der Atlantische Lachs, der Rotlachs, der Masu-Lachs, der Königslachs, der Ketalachs, der Silberlachs, der Donaulachs, der Pazifische Lachs und der Buckellachs dar.

Alternativ kann es sich auch um ein Futtermittel handeln, das zur Anzucht von Fischen dient, die anschließend zu Fischmehl oder Fischöl verarbeitet werden. Bei den Fischen handelt es sich hierbei vorzugsweise um Hering, Pollack, Menhaden, Anchovis, Kapelan oder Kabeljau. Das so erhaltene Fischmehl oder Fischöl kann wiederum in der Aquakultur zur Zucht von Speisefischen bzw. Crustaceen eingesetzt werden.

Die Aquakultur kann in Teichen, Tanks, Becken oder auch in abgegrenzten Arealen im Meer oder in Seen, hierbei insbesondere in Käfigen oder Netzpferchen, erfolgen. Die Aquakultur kann dazu dienen, den fertigen Speisefisch heranzuzüchten, jedoch auch eingesetzt werden, um Jungfische heranzuzüchten, die anschließend freigesetzt werden, um den Wildfischbestand aufzustocken.

Bei der Lachszucht erfolgt vorzugsweise zunächst die Heranzucht bis zum Junglachs in Süßwasser-Tanks oder künstlichen Wasserströmen und anschließend die weitere Zucht im Meer in schwimmenden Käfigen bzw. Netzpferchen, die vorzugsweise in Buchten oder Fjorden verankert sind.

Weiterer Gegenstand der vorliegenden Erfindung ist entsprechend auch ein Verfahren zum Züchten von Tieren, insbesondere Flossenfischen oder Crustaceen, vorzugsweise Lachs, bei welchem ein erfindungsgemäßes Futtermittel eingesetzt wird. Weiterer Gegenstand der vorliegenden Erfindung ist weiterhin ein Tier, insbesondere Flossenfisch oder Schalentier, das durch ein derartiges erfindungsgemäßes Verfahren erhältlich ist.

Verfahren zur Herstellung der Biomasse, insbesondere solcher Biomasse, die Lipide, insbesondere PUFAs, enthaltende Zellen, insbesondere der Ordnung Thraustochytriales, umfasst, sind im Stand der Technik ausführlich beschrieben. Die Herstellung erfolgt in der Regel dadurch, dass Zellen in Anwesenheit einer Kohlenstoffquelle und einer Stickstoffquelle in einem Fermenter kultiviert werden. Es können hierbei Biomasse-Dichten von mehr als 100 Gramm pro Liter und Produktionsraten von mehr als 0,5 Gramm Lipid pro Liter pro Stunde erreicht werden. Das Verfahren wird vorzugsweise als sogenanntes Fed-Batch-Verfahren durchgeführt, d.h. dass die Kohlenstoff- und Stickstoffquellen inkrementell während der Fermentation zugeführt werden. Die Lipid-Produktion kann nach Erreichen der gewünschten Biomasse durch unterschiedliche Maßnahmen induziert werden, beispielsweise durch Limitierung der Stickstoffquelle, der Kohlenstoffquelle oder des Sauerstoffgehalts oder Kombinationen davon.

Die Fermentation der Zellen erfolgt vorzugsweise in einem Medium mit niedriger Salinität, insbesondere um Korrosion zu verhindern. Dies kann dadurch erreicht werden, dass anstelle von Natriumchlorid chlor-freie Natriumsalze, wie beispielsweise Natriumsulfat, Natriumcarbonat, Natriumhydrogencarbonat oder Sodaasche, als Natriumquelle verwendet werden. Vorzugsweise wird Chlorid in Mengen von weniger als 3 g/l, insbesondere weniger als 500 mg/l, besonders bevorzugt weniger als 100 mg/l, bei der Fermentation eingesetzt.

Als Kohlenstoffquelle kommen sowohl alkoholische als auch nicht-alkoholische Kohlenstoffquellen in Betracht. Beispiele für alkoholische Kohlenstoffquellen sind Methanol, Ethanol und Isopropanol. Beispiele für nicht-alkoholische Kohlenstoffquellen sind Fructose, Glucose, Saccharose, Molassen, Stärke und Maissirup.

Als Stickstoffquelle kommen sowohl anorganische als auch organische Stickstoffquellen in Betracht. Beispiele für anorganische Stickstoffquellen sind Nitrate und Ammoniumsalze, insbesondere Ammoniumsulfat und Ammoniumhydroxid. Beispiele für organische Stickstoffquellen sind Aminosäuren, insbesondere Glutamat, und Harnstoff.

Zusätzlich können auch anorganische oder organische Phosphorverbindungen und/oder bekannte wachstumsstimulierende Stoffe, wie etwa Hefeextrakt oder Maisquellwasser, hinzugegeben werden, um die Fermentation positiv zu beeinflussen.

Erfindungsgemäß wird die Menge an während der Fermentation zugegebenem Sulfat so gewählt, dass sich in der resultierenden Biomasse in Bezug auf die Trockenmasse ein Sulfat-Gehalt von 25 bis 60 g/kg, insbesondere 25 bis 50, 25 bis 40 oder 25 bis 35 g/kg, einstellt.

Die Einstellung des Sulfat-Gehalts in der resultierenden Biomasse kann erfindungsgemäß auf unterschiedliche Weise erfolgen.

Beispielsweise kann in einem sogenannten Batch-Verfahren die benötigte Menge an Sulfat bereits zu Beginn vollständig vorgelegt werden. Die Menge an benötigtem Sulfat lässt sich einfach berechnen, da die zur Bildung der Biomasse eingesetzten Zellen das Sulfat fast vollständig assimilieren.

Bei Anwendung eines sogenannten Fed-Batch-Verfahrens kann alternativ die Menge an benötigten Sulfat im Verlauf der Fermentation zudosiert werden oder entsprechend ein Teil des Sulfats vorgelegt und der Rest im Verlauf der Fermentation zudosiert werden.

Insbesondere dann, wenn sich im Verlauf der Fermentation herausstellt, dass die Menge an produzierter Biomasse den ursprünglich kalkulierten Wert übertrifft, kann durch nachträgliches Zudosieren von Sulfat sichergestellt werden, dass die resultierenden Biomasse die bevorzugte Menge an Sulfat enthält.

Als Sulfat-Salz wird vorzugsweise Natriumsulfat, Ammoniumsulfat oder Magnesiumsulfat sowie Mischungen davon eingesetzt.

Der Chlorid-Gehalt liegt während der Fermentation, in Bezug auf das flüssige Fermentationsmedium einschließlich der enthaltenen Biomasse, vorzugsweise stets unter 3 g/kg, insbesondere unter 1 g/kg, besonders bevorzugt unter 400 mg/kg Fermentationsmedium.

Neben Sulfaten und gegebenenfalls eingesetzten Chloriden können bei der Fermentation gegebenenfalls auch weitere Salze, insbesondere ausgewählt aus Natriumcarbonat, Natriumhydrogencarbonat, Sodaasche oder anorganischen Phosphorverbindungen, eingesetzt werden.

Sofern weitere Salze eingesetzt werden, werden diese vorzugsweise in einer Menge eingesetzt, so dass jedes einzelne während der Fermentation, in Bezug auf das flüssige Fermentationsmedium einschließlich der enthaltenen Biomasse, jeweils in einer Menge von weniger als 10 g/kg, insbesondere weniger als 5 g/kg, besonders bevorzugt weniger als 3 g/kg im Fermentationsmedium vorliegt.

Der Gesamtsalzgehalt im Fermentationsmedium einschließlich der enthaltenen Biomasse liegt erfindungsgemäß vorzugsweise im Verlauf der gesamten Fermentation stets unter 35 g/kg, insbesondere unter 30 g/kg. Besonders bevorzugt liegt der Gesamtsalzgehalt während der gesamten Fermentation, in Bezug auf das flüssige Fermentationsmedium einschließlich der enthaltenen Biomasse, zwischen 10 und 35 kg/g, insbesondere zwischen 12 und 30 g/kg.

Der Sulfat-Gehalt im Fermentationsmedium einschließlich der enthaltenen Biomasse liegt erfindungsgemäß vorzugsweise im Verlauf der gesamten Fermentation stets zwischen 5 und 16 g/kg.

Unter "Sulfat-Gehalt" ist erfindungsgemäß der Gesamtgehalt an Sulfat, also der Gehalt an freiem und gebundenem, insbesondere organisch gebundenem, Sulfat zu verstehen. Es ist davon auszugehen, dass der Großteil des in der Biomasse enthaltenen Sulfats als Bestandteil von Exopolysacchariden vorliegt, die an der Ausbildung der Zellwand der Mikroorganismen beteiligt sind.

Die Bestimmung des Sulfat-Gehalts erfolgt erfindungsgemäß durch Ermittlung des Schwefel-Gehalts der erhaltenen Biomasse, da der Großteil des in der Biomasse enthaltenen Schwefels auf das enthaltene Sulfat zurückzuführen ist. Schwefel, der auf andere Quellen zurückzuführen ist, ist aufgrund der Menge des enthaltenen Sulfats zu vernachlässigen. Aus der Menge des ermittelten Schwefels kann somit ohne Weiteres die Menge an enthaltenem Sulfat ermittelt werden.

Der Schwefel-Gehalt der Biomasse wird hierbei durch Elementaranalyse gemäß DIN EN ISO 11885 bestimmt. Für die Analyse des Schwefelgehalts der Biomasse werden entsprechende Aliquote der Probe vor der Analyse vorzugsweise mit Salpetersäure und Wasserstoffperoxid bei 240°C unter Druck aufgeschlossen, um die freie Zugänglichkeit des enthaltenen Schwefels sicherzustellen.

Erfindungsgemäß wird daher vorzugsweise zur Herstellung des Futtermittels eine PUFAs enthaltende Biomasse eingesetzt, die sich dadurch auszeichnet, dass sich in dieser durch Elementaranlyse gemäß DIN EN ISO 11885 ein Schwefel-Gehalt von 8 bis 20 g/kg, bezogen auf die Trockenmasse, nachweisen lässt. Der Schwefel-Gehalt in der Biomasse beträgt hierbei vorzugsweise 8 bis 17 g/kg, insbesondere 8 bis 14 g/kg, besonders bevorzugt 8 bis 12 g/kg, jeweils bezogen auf die Trockenmasse.

Der Phosphor-Gehalt erfindungsgemäß bevorzugt eingesetzter Biomassen beträgt erfindungsgemäß, in Bezug auf die Trockenmasse, vorzugsweise 1 bis 6 g/kg, insbesondere 2 bis 5 g/kg. Der Phosphor-Gehalt wird vorzugsweise ebenfalls durch Elementaranalyse gemäß DIN EN ISO 11885 ermittelt.

Die Fermentation der Zellen erfolgt vorzugsweise bei einem pH-Wert von 3 bis 11, insbesondere 4 bis 10, sowie vorzugsweise bei einer Temperatur von mindestens 20°C, insbesondere 20 bis 40°C, besonders bevorzugt mindestens 30 °C. Ein typisches Fermentationsverfahren dauert bis zu etwa 100 Stunden.

Die Zellen werden erfindungsgemäß vorzugsweise bis zu einer Biomassendichte von mindestens 50, 60 oder 70 g/l, insbesondere mindestens 80 oder 90 g/l, besonders bevorzugt mindestens 100 g/l, fermentiert. Die Angaben beziehen sich hierbei auf den Gehalt an Bio-Trockenmasse in Bezug auf das Gesamtvolumen der Fermentationsbrühe nach Beendigung der Fermentation. Der Gehalt an Bio-Trockenmasse wird bestimmt durch Abfiltrieren der Biomasse aus der Fermentationsbrühe, anschließendes Waschen mit Wasser, danach vollständige Trocknung - beispielsweise in der Mikrowelle - und schließlich Feststellung des Trockengewichts.

Nach Beendigung der Fermentation erfolgt die Ernte der Biomasse. Vorzugsweise erfolgt nach der Ernte der Biomasse oder gegebenenfalls auch schon kurz vor der Ernte der Biomasse eine Pasteurisierung der Zellen, um die Zellen abzutöten und Enzyme, die den Abbau der Lipide befördern könnten, zu inaktivieren. Die Pasteurisierung erfolgt vorzugsweise durch Erhitzen der Biomasse auf eine Temperatur von 50 bis 121 °C für eine Dauer von 5 bis 60 Minuten.

Ebenso erfolgt nach der Ernte der Biomasse oder gegebenenfalls auch schon kurz vor der Ernte der Biomasse vorzugsweise die Zugabe von Antioxidantien, um den in der Biomasse enthaltenen Wertstoff vor oxidativem Abbau zu schützen. Bevorzugte Antioxidantien stellen hierbei BHT, BHA, TBHA, Ethoxyquin, beta-Carotin, Vitamin E und Vitamin C dar. Das Antioxidans wird, sofern eingesetzt, vorzugsweise in einer Menge von 0,01 bis 2 Gew.-% hinzugegeben.

Gegebenenfalls kann nun, vor der eigentlichen Trocknung, bereits ein Teil des Fermentationsmediums von der Biomasse abgetrennt und damit der Feststoffanteil erhöht werden. Dies kann insbesondere durch Zentrifugation, Flotation, Filtration, insbesondere Ultra- oder Mikrofiltration, Dekantieren und/oder Lösungsmittelverdampfung erfolgen. Die Lösungsmittelverdampfung erfolgt hierbei vorzugsweise unter Einsatz eines Rotationsverdampfers, eines Dünnschichtverdampfers oder eines Fallfilmverdampfers in einem einstufigen oder mehrstufigen Prozess. Alternativ kommt beispielsweise auch die Umkehrosmose zwecks Einengung der Fermentationsbrühe in Betracht.

In diesem ersten optionalen, aber bevorzugt durchgeführten, Schritt erfolgt vorzugsweise eine Aufkonzentrierung der Fermentationsbrühe auf einen Feststoffgehalt von mindestens 10 oder 15 Gew.-%, vorzugsweise von mindestens 20 oder 25 Gew.-%, insbesondere 10 bis 50 oder 15 bis 45 Gew.-%, besonders bevorzugt 15 bis 40 Gew.-% oder 20 bis 40 Gew.-%.

D.h. die in einem erfindungsgemäßen Verfahren zu trocknende Biomasse liegt vorzugsweise in Form einer Suspension mit dem zuvor angegebenen Feststoffanteil vor, wobei es sich bei der Suspension vorzugsweise um Fermentationsbrühe oder eingeengte Fermentationsbrühe handelt.

Nach der optionalen Einengung der Fermentationsbrühe erfolgt nun die erfindungsgemäße Trocknung der Biomasse, vorzugsweise durch Sprühtrocknung, insbesondere Düsensprühtrocknung, Sprühgranulation, Wirbelschichtgranulation, insbesondere Fließbettgranulation, oder in einem Trommeltrockner.

Alternativ kann die Biomasse auch direkt nach der Ernte ohne vorherige Einengung dem Trocknungsschritt unterzogen werden, insbesondere dann, wenn die erhaltene Fermentationsbrühe bereits einen hohen Feststoffgehalt, vorzugsweise wie zuvor angegeben, aufweist.

Durch die Trocknung der Biomasse wird diese vorzugsweise auf eine Restfeuchte von maximal 10 Gew.-%, insbesondere 0 bis 10 Gew.-%, besonders bevorzugt maximal 8 Gew.-%, insbesondere 0,5 bis 8 Gew.-%, vor allem maximal 6 oder 5 Gew.-%, insbesondere 0,5 bis 6 oder 0,5 bis 5 Gew.-%, getrocknet.

Unter "Trockenmasse" ist erfindungsgemäß entsprechend vorzugsweise ein Produkt zu verstehen, die einen Feuchtigkeitsgehalt von unter 10 Gew.-%, insbesondere unter 5 Gew.-%, aufweist.

Durch die Trocknung wird vorzugweise ein rieselfähiges, feinteiliges oder grobkörniges Produkt, vorzugsweise Granulat, erhalten. Gegebenenfalls kann aus dem erhaltenen Granulat durch Sieben oder Staubabtrennung ein Produkt mit der gewünschten Korngröße erhalten werden.

Sofern ein rieselfähiges feinteiliges Pulver erhalten wurde, kann dieses gegebenenfalls durch geeignete Kompaktier- oder Granulierverfahren in ein grobkörniges, gut rieselfähiges, lagerbares und weitgehend staubfreies Produkt überführt werden.

Bei dieser anschließenden Granulation oder Kompaktierung können gegebenenfalls übliche organische oder anorganische Hilfsstoffe beziehungsweise Träger wie Stärke, Gelatine, Cellulosederivate oder ähnliche Stoffe, die üblicherweise in der Lebensmittel- oder Futterverarbeitung als Binde-, Gelier- oder Verdickungsmittel Verwendung finden, eingesetzt werden.

Unter "rieselfähig" ist erfindungsgemäß ein Pulver zu verstehen, das aus einer Serie von Glasauslaufgefäßen mit verschieden großen Auslassöffnungen mindestens aus dem Gefäß mit der Öffnung 5 Millimeter ungehindert auslaufen kann (Klein: Seifen, Öle, Fette, Wachse 94, 12 (1968)).

Unter "feinteilig" ist erfindungsgemäß ein Pulver mit einem überwiegenden Anteil (> 50 %) einer Korngröße von 20 bis 100 Mikrometer Durchmesser zu verstehen.

Unter "grobkörnig" ist erfindungsgemäß ein Pulver mit einem überwiegenden Anteil (>50 %) einer Korngröße von 100 bis 2500 Mikrometer Durchmesser zu verstehen.

Unter "staubfrei" ist erfindungsgemäß ein Pulver zu verstehen, das lediglich geringe Anteile (< 10 %, vorzugsweise < 5 %) an Korngrößen unter 100 Mikrometer enthält.

Die Bestimmung der Korn- bzw. Teilchengrößen erfolgt erfindungsgemäß vorzugsweise durch Methoden der Laserbeugungsspektrometrie. Die anzuwendenden Methoden sind im Lehrbuch "Teilchengrößenmessung in der Laborpraxis" von R.H. Müller und R. Schuhmann, Wissenschaftliche Verlagsgesellschaft Stuttgart (1996) sowie im Lehrbuch "Introduction to Particle Technology" von M. Rhodes, Verlag Wiley & Sons (1998) beschrieben. Sofern unterschiedliche Methoden anwendbar sind, wird bevorzugt die zuerst genannte anwendbare Methode aus dem Lehrbuch von R.H. Müller und R. Schuhmann zur Teilchengrößenmessung angewendet.

Die durch Trocknung erhaltene Biomasse besitzt vorzugsweise einen Anteil von mindestens 80 Gew.-%, insbesondere mindestens 90 Gew.-%, besonders bevorzugt mindestens 95 Gew.-%, an Partikeln mit einer Korngröße von 100 bis 3500 Mikrometern, vorzugsweise 100 bis 3000 Mikrometern, vor allem 100 bis 2500 Mikrometern.

**Ausführungsbeispiele**

*Beispiel 1: Herstellung der Biomasse durch Fermentation von Aurantiochytrium limacinum SR21 in einem Medium mit hohem Sulfat-Gehalt und anschließende Trocknung der Biomasse*

Die Kultivierung der Zellen erfolgte für ca. 75 h in einem Zulaufverfahren unter Verwendung eines Stahlfermenters mit einem Fermentervolumen von 2 Litern bei einer Gesamt-Startmasse von 712 g und einer erreichten Gesamt-Endmasse von 1,3 - 1,5 kg. Während des Verfahrens wurde eine Glucose-Lösung (570 g/kg Glucose) zudosiert ("Fedbatch Verfahren")

Die Zusammensetzung des Startmediums war wie folgt:
Medium 1: 20 g/kg Glukose; 4 g/kg Hefe-Extrakt; 16 g/kg Natriumsulfat; 2 g/kg Ammoniumsulfat; 2,46 g/kg Magnesiumsulfat (Heptahydrat); 0,45 g/kg Kaliumchlorid; 4,5 g/kg Kaliumdihydrogenphosphat; 0,1 g/kg Thiamin (HCl); 5 g/kg Spurenelementlösung.

Die Zusammensetzung der Spurenelementlösung war wie folgt: 35 g/kg Salzsäure (37%); 1,86 g/kg Manganchlorid (Tetrahydrat); 1,82 g/kg Zinksulfat (Heptahydrat); 0,818 g/kg Natrium-EDTA; 0,29 g/kg Borsäure; 0,24 g/kg Natriummolybdat (Dihydrat); 4,58 g/kg Kalziumchlorid (Dihydrat); 17,33 g/kg Eisensulfat (Heptahydrat); 0,15 g/kg Kupferchlorid (Dihydrat).

Die Kultivierung erfolgte unter folgenden Bedingungen: Kultivierungstemperatur 28°C; Belüftungsrate 0,5 vvm, Rührergeschwindigkeit 600 - 1950 rpm, Kontrolle das pH-Werts in der Wachstumsphase bei 4,5 mit Hilfe von Ammoniakwasser (25% v/v). Die Fermentation erfolgte bis zu einer Biomassendichte von 116 g/l.

Nach der Kultivierung wurden die Fermentationsbrühen für 20 Minuten auf 60°C erhitzt um eine weitere Aktivität der Zellen zu unterbinden.

Anschließend erfolgte eine zweistufige Trocknung der Biomasse: Zunächst wurde die Fermentationsbrühe durch Verdampfung auf eine Trockenmasse von etwa 20 Gew.-% eingeengt. Anschließend erfolgte Sprühtrocknung der eingeengten Fermentationsbrühe unter Verwendung eines Production Minor™ Spray Dryer (GEA NIRO) bei einer Einlasstemperatur der Trocknungsluft von 340°C. Durch Sprühtrocknung wurde so ein Puder mit einer Trockenmasse von mehr als 95 Gew.-% erhalten.

Zur Bestimmung des Sulfat-Gehalts der erhaltenen Biomasse wurde der Schwefel-Gehalt der Biomasse gemäß DIN ISO 11885 bestimmt. Hierzu wurde ein Aliquot der Biomasse zunächst mit Salpetersäure und Wasserstoffperoxid bei 240°C unter Druck aufgeschlossen. Der ermittelte Schwefelgehalt lag bei 11 g/kg Biomasse, was einem Sulfat-Gehalt von 33 g/kg Biomasse entspricht.

*Beispiel 2: Futtermittel-Herstellung durch Extrusion*

Es wurden die in Tabelle 1 dargestellten Futtermittel-Mischungen hergestellt. Neben der erfindungsgemäß einzusetzenden Biomasse gemäß Beispiel 1 wurden zwei weitere handelsübliche Labyrinthulea-Biomassen sowie Fischöl als momentan noch übliche Quelle für omega-3-Fettsäuren zum Vergleich getestet.

Die Herstellung der Futtermittel-Mischungen erfolgte jeweils durch Vermischung der Komponenten - mit Ausnahme der Öle - unter Verwendung eines Doppelhelix-Mixers (Modell 500L, TGC Extrusion, Frankreich). Die so erhaltenen Mischungen wurden anschließend unter Verwendung einer Hammermühle (Modell SH1, Hosokawa-Alpine, Deutschland) auf Teilchengrößen unterhalb 250 μm zerkleinert.

Tabelle 1: In der Extrusion eingesetzte Futtermittelzusammensetzungen (Angaben in Gew.-%)

| Inhaltsstoff | M1 | M2 | M3 | M4 |
|---|---|---|---|---|
| Fischmehl | 10,00 | 10,00 | 10,00 | 10,00 |
| Sojaprotein-Konzentrat | 23,10 | 23,20 | 23,10 | 20,27 |

(fortgesetzt)

| Inhaltsstoff | M1 | M2 | M3 | M4 |
|---|---|---|---|---|
| Erbsenprotein-Konzentrat | 15,00 | 15,00 | 15,00 | 15,00 |
| Weizen gluten | 9,90 | 9,90 | 9,90 | 9,90 |
| Weizenmehl | 18,12 | 10,82 | 10,55 | 16,46 |
| Fischöl | 10,00 | -- | -- | -- |
| Biomasse aus Beispiel 1 | -- | 16,00 | -- | -- |
| Handelsübliche Biomasse 1 | -- | -- | 16,74 | -- |
| Handelsübliche Biomasse 2 | -- | -- | -- | 13,52 |
| Rapsöl | 10,00 | 11,00 | 11,00 | 11,00 |
| Vitamin/Mineral-Prämix | 1,00 | 1,00 | 1,00 | 1,00 |
| Dicalciumphosphat | 2,00 | 2,00 | 2,00 | 2,00 |
| Yttriumoxid | 0,03 | 0,03 | 0,03 | 0,03 |
| DL-Methionin | 0,35 | 0,36 | 0,33 | 0,33 |
| Aquavi-Lys | 0,17 | 0,35 | 0,08 | 0,19 |
| Tryp-Amino | 0,09 | 0,09 | 0,08 | 0,09 |
| L-Histidin | 0,24 | 0,25 | 0,19 | 0,21 |

Für die Extrusion wurden jeweils 140 kg pro Futtermittel eingesetzt. Die Extrusion wurde mittels eines Doppelschneckenextruders (CLEXTRAL BC45) mit einem Schneckendurchmesser von 55,5 mm und einer maximalen Fließrate von 90-100 kg/h durchgeführt. Es wurden Pellets mit einer Größe von 4,0 mm extrudiert. Der Extruder wurde hierzu mit einem Hochgeschwindigkeits-Cutter ausgestattet, um das Produkt in die vorgesehene Pellet-Größe zu überführen.

Es wurden nun verschiedene Extrusionsparameter getestet, um herauszufinden, unter welchen Extrusions-Bedingungen eine optimale Ölbeladungskapazität des erhaltenen Extrudats erhalten werden kann. Es hat sich hierbei überraschenderweise herausgestellt, dass eine optimale Ölbeladungskapazität mit einem sehr niedrigen Energieeintrag erreicht werden kann. Der Energieeintrag war hierbei deutlich geringer als bei der Verwendung von Fischöl. Weiterhin war der optimale Energieeitrag bei einer erfindungsgemäß vorzugsweise zu verwendenden Biomasse mit hohem Sulfat-Gehalt nochmal deutlich geringer als bei handelsüblichen Thraustochytriales-Biomassen. Die Ergebnisse sind in Tabelle 2 dargestellt.

Tabelle 2: Energieeintrage zur Herstellung von Pellets mit der gewünschten Ölbeladungskapazität

| Diät | Barrel 1 Temp (°C) | Barrel 2 Temp (°C) | Feeder-Rate (kg/h) | Drehgeschwindigkeit (rpm) | Wassermenge (0-10) | Stromstärke (A) | SME (Wh/kg) |
|---|---|---|---|---|---|---|---|
| M1 | 31 | 116-118 | 112 | 215 | 9 | 11 | 34,6 |
| M2 | 32 | 98-104 | 141 | 253 | 5 | 7 | 20,6 |
| M3 | 32 | 97-102 | 136 | 255 | 5 | 8 | 24,6 |
| M4 | 31 | 99-107 | 133 | 253 | 5 | 8 | 24,9 |

Bei der Größe "SME" handelt es sich hierbei um die spezifische mechanische Energie. Diese wird wie folgt berechnet:

$$SME\ (W\,h/kg) = \frac{U \times I \times \cos\Phi \; \frac{Test\,SS}{Max\,SS}}{Qs}$$

mit

U: Arbeitsspannung des Motors (vorliegend 460 V)

I: Stromstärke des Motors (A)

cos Φ: theoretische Leistung des Extrudermotors (vorliegend 0,95)

Test SS: Test-Geschwindigkeit (rpm) der rotierenden Schnecken

Max SS: Maximal-Geschwindigkeit (267 rpm) der rotierenden Schnecken

Qs: Einlass-Fließrate des Futterbreis (kg/h)

Nach der Extrusion wurde das Extrudat in einem vibrierenden Fließbett-Trockner (Modell DR100, TGC Extrusion, Frankreich) getrocknet.

Abschließend erfolgte nach dem Abkühlen des Extrudats eine Öl-Beschichtung durch Vakuumbeschichtung (Vakuumbeschichter PG-10VCLAB, Dinnisen, Niederlande). Es wurde hierbei festgestellt, dass mehr als 0,35 g Öl auf 1 g Extrudat aufgetragen werden können.

*Beispiel 3: Ermittlung der Abriebfestigkeit und Wasserstabilität*

Die Abriebfestigkeit wurde auf folgende Weise ermittelt: Das getrocknete Extrusionsprodukt wurde vor der Beladung mit Öl einer mechanischen Belastung unter Verwendung des Holmen Pellet-Tester (Borregaard Lignotech, Hull, UK) ausgesetzt. Vor Durchführung des Tests wurden die Proben gesiebt, um möglicherweise anhaftende Feinteilchen zu entfernen. Die aufbereiteten Proben (100 g) wurden anschließend in den Pellet-Tester eingeführt unter Verwendung eines 2,5 mm-Filtersiebs. Die Pellets wurden anschließend mit hoher Luftgeschwindigkeit für 30 Sekunden durch ein Röhrchen befördert, das rechtwinklige Rohrbogen aufwies. Anschließend wurde der Abrieb durch Wiegen bestimmt. Die Abriebfestigkeit wurde als PDI (Pellet Durability Index) angegeben, definiert als die Menge an auf dem Filtersieb verbliebener Probe in Prozent. Der Test wurde jeweils mit drei Proben durchgeführt und anschließend der Mittelwert gebildet.

Die Wasserstabilität wurde mit den ölbeladenen Proben durchgeführt. Die Methode wurde im Wesentlichen ausgeführt wie durch Baeverfjord et al. (2006; Aquaculture 261, 1335-1345) beschrieben, mit geringen Modifikationen. 10 g-Proben wurden in metallische Infusions-Körbe mit einer Maschenweite von 0,3 mm gegeben. Die Infusions-Körbe wurden anschließend in eine Plastik-Wanne mit Wasser gegeben, so dass die Proben vollständig mit Wasser bedeckt waren. Die Wanne wurde anschließend für 30 Minuten einer Schüttelagitation von 30 Schütteleinheiten pro Minute ausgesetzt. Danach wurden die Proben vorsichtig mit Löschpapier getrocknet und anschließend gewogen bevor und nachdem sie einer Ofen-Trocknung bei einer Temperatur von 105 °C für 24 Stunden unterzogen worden waren. Die Wasserstabilität wurde berechnet als die Differenz des Trockengewichts der Probe vor und nach der Inkubation in Wasser und in Prozent des Trockengewichts der eingesetzten Probe vor der Inkubation mit Wasser angegeben.

Die Ergebnisse sind in Tabelle 3 dargestellt.

| Probe | M1 | M2 | M3 | M4 |
|---|---|---|---|---|
| Abriebfestigkeit [%] | 90,0 | 93,3 | 88,3 | 85,2 |
| Wasserstabilität [%] | 95,7 | 98,5 | 93,8 | 90,2 |

Es ist zu erkennen, dass ein erfindungsgemäßes Futtermittel eine deutlich höhere Abriebfestigkeit und Wasserstabilität aufweist als Futtermittel, die eine auf dem Markt erhältliche Labyrinthulea-Biomasse oder Fischöl als Quelle für omega-3-Fettsäuren enthalten.

**Patentansprüche**

1.   Verfahren zur Herstellung eines PUFAs enthaltenden Futtermittels, **dadurch gekennzeichnet, dass** eine PUFAs enthaltende Biomasse des Taxons Labyrinthulomycetes, die einen Sulfat-Gehalt, bezogen auf die Trockenmasse, von 25 bis 60 g/kg aufweist, mit weiteren Futtermittelkomponenten bei einem Energieeintrag von 12 - 28 Wh/kg extrudiert wird, wobei unter Trockenmasse ein Produkt mit einer Restfeuchte von unter 5 Gew.% zu verstehen ist und wobei der Sulfat-Gehalt der Biomasse über den Gehalt an Schwefel in der Biomasse und dieser wiederum nach DIN EN ISO 11885 ermittelt wird.

2. Verfahren nach Anspruch 1, **dadurch gekennzeichnet, dass** die Extrusion bei einem Energieeintrag von 14-26, vorzugsweise 16-24, insbesondere 18-22 Wh/kg, erfolgt.

3. Verfahren nach Anspruch 1 oder 2, **dadurch gekennzeichnet, dass** es sich bei der PUFAs enthaltenden Biomasse des Taxons Labyrinthulomycetes um eine solche der Familie der Thraustochytriaceae, besonders bevorzugt der Gattungen Thraustochytrium, Schizochytrium, Aurantiochytrium, Oblongichytrium oder Ulkenia, vor allem der Gattung Aurantiochytrium, handelt.

4. Verfahren nach Anspruch 3, **dadurch gekennzeichnet, dass** es sich bei der PUFAs enthaltenden Biomasse um eine der Spezies Aurantiochytrium limacinum, insbesondere des Stamms Aurantiochytrium limacinum SR21, handelt.

5. Verfahren nach einem der vorhergehenden Ansprüche, **dadurch gekennzeichnet, dass** die Biomasse einen Sulfat-Gehalt, bezogen auf die Trockenmasse, von 25 bis 50 g/kg, bevorzugt 25 bis 40, vor allem 25 bis 35 g/kg, aufweist.

6. Verfahren nach einem der vorhergehenden Ansprüche, **dadurch gekennzeichnet, dass** die bei der Extrusion eingesetzte Zusammensetzung folgende Eigenschaften aufweist:

   a) einen Gesamt-Proteingehalt von 33 bis 67 Gew.-%, vorzugsweise 39 bis 61 Gew.-%, insbesondere 44 bis 55 Gew.-%;
   b) einen Gesamt-Fettgehalt von 5 bis 25 Gew.-%, vorzugsweise 8 bis 22 Gew.-%, insbesondere 10 bis 20 Gew.-%, vor allem 12 bis 18 Gew.-%;
   c) einen Gesamt-Stärkegehalt von maximal 25 Gew.-%, insbesondere maximal 20 Gew.-%, vorzugsweise 6 bis 17 Gew.-%, besonders bevorzugt 8 bis 14 Gew.-%;
   d) einen Gehalt an Labyrinthulomycetes-Biomasse, bevorzugt Thraustochytriaceae-Biomasse, von 2 bis 24 Gew.-%, vorzugsweise 4 bis 22 Gew.-%, insbesondere 9 bis 20 Gew.-%, vor allem 11 bis 18 Gew.-%;
   e) vorzugsweise einen Gehalt an polyungesättigten Fettsäuren (PUFAs) von 0,8 bis 8 Gew.-%, vorzugsweise 1,2 bis 6 Gew.-%, insbesondere 1,4 bis 5 Gew.-%, vor allem 1,5 bis 4 Gew.-%;
   f) vorzugsweise einen Gehalt an omega-3-Fettsäuren von 0,8 bis 8 Gew.-%, vorzugsweise 1,2 bis 6 Gew.-%, insbesondere 1,4 bis 5 Gew.-%, vor allem 1,5 bis 4 Gew.-%;
   g) vorzugsweise Gehalt an DHA von 0,1 bis 4,0 Gew.-%, vorzugsweise 0,25 bis 3,0 Gew.-%, insbesondere 0,5 bis 2,8 Gew.-%, vor allem 0,8 bis 2,5 Gew.-%, insbesondere 1,0 bis 2,0 Gew.-%.

7. Verfahren nach einem der vorhergehenden Ansprüche, **dadurch gekennzeichnet, dass** nach der Extrusion und gegebenenfalls Trocknung des Extrudats eine Beschichtung des Extrudats mit Öl, insbesondere pflanzlichem Öl, vorzugsweise in einer Menge von, in Bezug auf das finale Produkt, 3 bis 17 Gew.-%, insbesondere 5 bis 15 Gew.-%, erfolgt.

8. PUFAs enthaltendes Futtermittel-Extrudat, das eine PUFAs enthaltende Biomasse des Taxons Labyrinthulomycetes umfasst, **dadurch gekennzeichnet, dass** die Biomasse einen Sulfat-Gehalt, bezogen auf die Trockenmasse, von 25 bis 60 g/kg aufweist und dass das Extrudat eine Öl-Beladungskapazität von mindestens 0,25 g Öl pro g Extrudat, vorzugsweise mindestens 0,30 g Öl pro g Extrudat, besonders bevorzugt mindestens 0,35 g Öl pro g Extrudat, aufweist, wobei unter Trockenmasse ein Produkt mit einer Restfeuchte von unter 5 Gew.% zu verstehen ist und wobei der Sulfat-Gehalt der Biomasse über den Gehalt an Schwefel in der Biomasse und dieser wiederum nach DIN EN ISO 11885 ermittelt wird.

9. Futtermittel-Extrudat nach vorhergehendem Anspruch, **dadurch gekennzeichnet, dass** es sich bei der PUFAs enthaltenden Biomasse des Taxons Labyrinthulomycetes um eine Biomasse der Familie der Thraustochytriaceae, besonders bevorzugt der Gattungen Thraustochytrium, Schizochytrium, Aurantiochytrium, Oblongichytrium oder Ulkenia, vor allem der Gattung Aurantiochytrium, handelt.

10. Futtermittel-Extrudat nach vorhergehendem Anspruch, **dadurch gekennzeichnet, dass** es sich bei der Biomasse um eine der Spezies Aurantiochytrium limacinum, insbesondere des Stamms Aurantiochytrium limacinum SR21, handelt.

**11.** Futtermittel-Extrudat nach einem der vorhergehenden Ansprüche, **dadurch gekennzeichnet, dass** die Biomasse einen Sulfat-Gehalt, bezogen auf die Trockenmasse, von 25-50 g/kg, bevorzugt 25 bis 40, vor allem 25 bis 35 g/kg, aufweist.

**12.** PUFAs enthaltendes Futtermittel-Extrudat nach einem der vorhergehenden Ansprüche, **dadurch gekennzeichnet, dass** es folgende Eigenschaften aufweist:

a) einen Gesamt-Proteingehalt von 30 bis 60 Gew.-%, vorzugsweise 35 bis 55 Gew.-%, insbesondere 40 bis 50 Gew.-%;
b) einen Gesamt-Fettgehalt von 15 bis 35 Gew.-%, vorzugsweise 18 bis 32 Gew.-%, insbesondere 20 bis 30 Gew.-%, vor allem 22 bis 28 Gew.-%;
c) einen Gesamt-Stärkegehalt von maximal 25 Gew.-%, insbesondere maximal 20 Gew.-%, vorzugsweise 5 bis 15 Gew.-%, besonders bevorzugt 7 bis 13 Gew.-%;
d) einen Gehalt an Labyrinthulomycetes-Biomasse, vorzugsweise an Thraustochytriaceae-Biomasse, von 2 bis 22 Gew.-%, vorzugsweise 4 bis 20 Gew.-%, insbesondere 8 bis 18 Gew.-%, vor allem 10 bis 16 Gew.-%;
e) vorzugsweise einen Gehalt an polyungesättigten Fettsäuren (PUFAs) von 2 bis 12 Gew.-%, vorzugsweise 3 bis 10 Gew.-%, insbesondere 4 bis 9 Gew.-%, vor allem 5 bis 8 Gew.-%;
f) vorzugsweise einen Gehalt an omega-3-Fettsäuren von 1 bis 6 Gew.-%, vorzugsweise 1,5 bis 5 Gew.-%, insbesondere 2 bis 4,5 Gew.-%, vor allem 2,5 bis 4 Gew.-%;
g) vorzugsweise einen Gehalt an DHA von 0,5 bis 3 Gew.-%, vorzugsweise 0,8 bis 2,5 Gew.-%, insbesondere 1 bis 2,5 Gew.-%, vor allem 1,2 bis 2,2 Gew.-%, insbesondere 1,2 bis 2,0 Gew.-%.

**13.** Verfahren zur Anzucht von Tieren, insbesondere Fischen, bei welchem ein Futtermittel-Extrudat nach einem der vorhergehenden Ansprüche zum Einsatz kommt.

## Claims

**1.** Process for the preparation of a PUFAs-comprising feedstuff, **characterized in that** a PUFAs-comprising biomass of the taxon Labyrinthulomycetes that has a sulfate content of 25 to 60 g/kg, based on the dry matter, is extruded together with further feedstuff components at an energy input of 12 - 28 Wh/kg, wherein dry matter is to be understood as meaning a product having a residual moisture content of below 5% by weight and wherein the sulfate content of the biomass is determined via the sulfur content in the biomass and this in turn is determined according to DIN EN ISO 11885.

**2.** Process according to Claim 1, **characterized in that** the extrusion takes place at an energy input of 14 - 26, preferably 16 - 24, in particular 18 - 22 Wh/kg.

**3.** Process according to Claim 1 or 2, **characterized in that** the PUFAs-comprising biomass of the taxon Labyrinthulomycetes is that of the family Thraustochytriaceae, especially preferably of the genera Thraustochytrium, Schizochytrium, Aurantiochytrium, Oblongichytrium or Ulkenia, especially the genus Aurantiochytrium.

**4.** Process according to Claim 3, **characterized in that** the PUFAs-comprising biomass is a biomass of the species Aurantiochytrium limacinum, in particular of the strain Aurantiochytrium limacinum SR21.

**5.** Process according to one of the preceding claims, **characterized in that** the biomass has a sulfate content of 25 to 50 g/kg, preferably 25 to 40, especially 25 to 35 g/kg, based on the dry matter.

**6.** Process according to one of the preceding claims, **characterized in that** the composition employed in the extrusion has the following properties:

a) a total protein content of 33 to 67% by weight, preferably 39 to 61% by weight, in particular 44 to 55% by weight;
b) a total fat content of 5 to 25% by weight, preferably 8 to 22% by weight, in particular 10 to 20% by weight, especially 12 to 18% by weight;
c) a total starch content of not more than 25% by weight, in particular not more than 20% by weight, preferably 6 to 17% by weight, especially preferably 8 to 14% by weight;
d) a Labyrinthulomycetes biomass content, preferably Thraustochytriaceae biomass content, of 2 to 24% by weight, preferably 4 to 22% by weight, in particular 9 to 20% by weight, especially 11 to 18% by weight;

e) preferably a polyunsaturated fatty acids (PUFAs) content of 0.8 to 8% by weight, preferably 1.2 to 6% by weight, in particular 1.4 to 5% by weight, especially 1.5 to 4% by weight;

f) preferably an omega-3 fatty acids content of 0.8 to 8% by weight, preferably 1.2 to 6% by weight, in particular 1.4 to 5% by weight, especially 1.5 to 4% by weight;

g) preferably a DHA content of 0.1 to 4.0% by weight, preferably 0.25 to 3.0% by weight, in particular 0.5 to 2.8% by weight, especially 0.8 to 2.5% by weight, in particular 1.0 to 2.0% by weight.

7. Process according to one of the preceding claims, **characterized in that**, after extrusion and, if appropriate, drying of the extrudate, the extrudate is coated with oil, in particular vegetable oil, preferably in an amount of 3 to 17% by weight, in particular 5 to 15% by weight, based on the final product.

8. PUFAs-comprising feedstuff extrudate comprising a PUFAs-comprising biomass of the taxon Labyrinthulomycetes, **characterized in that** the biomass has a sulfate content of 25 to 60 g/kg, based on the dry matter, and **in that** the extrudate has an oil load capacity of at least 0.25 g of oil per g of extrudate, preferably at least 0.30 g of oil per g of extrudate, especially preferably at least 0.35 g of oil per g of extrudate, wherein dry matter is to be understood as meaning a product having a residual moisture content of below 5% by weight and wherein the sulfate content of the biomass is determined via the sulfur content in the biomass and this in turn is determined according to DIN EN ISO 11885.

9. Feedstuff extrudate according to the preceding claim, **characterized in that** the PUFAs-comprising biomass of the taxon Labyrinthulomycetes is a biomass of the family Thraustochytriaceae, especially preferably of the genera Thraustochytrium, Schizochytrium, Aurantiochytrium, Oblongichytrium or Ulkenia, especially the genus Aurantiochytrium.

10. Feedstuff extrudate according to the preceding claim, **characterized in that** the biomass is a biomass of the species Aurantiochytrium limacinum, in particular of the strain Aurantiochytrium limacinum SR21.

11. Feedstuff extrudate according to one of the preceding claims, **characterized in that** the biomass has a sulfate content of 25 - 50 g/kg, preferably 25 to 40, especially 25 to 35 g/kg, based on the dry matter.

12. PUFAs-comprising feedstuff extrudate according to one of the preceding claims, **characterized in that** it has the following properties:

a) a total protein content of 30 to 60% by weight, preferably 35 to 55% by weight, in particular 40 to 50% by weight;

b) a total fat content of 15 to 35% by weight, preferably 18 to 32% by weight, in particular 20 to 30% by weight, especially 22 to 28% by weight;

c) a total starch content of not more than 25% by weight, in particular not more than 20% by weight, preferably 5 to 15% by weight, especially preferably 7 to 13% by weight;

d) a Labyrinthulomycetes biomass content, preferably Thraustochytriaceae biomass content, of 2 to 22% by weight, preferably 4 to 20% by weight, in particular 8 to 18% by weight, especially 10 to 16% by weight;

e) preferably a polyunsaturated fatty acids (PUFAs) content of 2 to 12% by weight, preferably 3 to 10% by weight, in particular 4 to 9% by weight, especially 5 to 8% by weight;

f) preferably an omega-3 fatty acids content of 1 to 6% by weight, preferably 1.5 to 5% by weight, in particular 2 to 4.5% by weight, especially 2.5 to 4% by weight;

g) preferably a DHA content of 0.5 to 3% by weight, preferably 0.8 to 2.5% by weight, in particular 1 to 2.5% by weight, especially 1.2 to 2.2% by weight, in particular 1.2 to 2.0% by weight.

13. Method of growing animals, in particular fish, in which a feedstuff extrudate according to one of the preceding claims is employed.

**Revendications**

1. Procédé pour la préparation d'un aliment pour animaux contenant des AGPI, **caractérisé en ce qu'**une biomasse contenant des AGPI du taxon Labyrinthulomycetes, qui présente une teneur en sulfate, par rapport à la masse sèche, de 25 à 60 g/kg, est extrudée avec d'autres composants d'aliment pour animaux avec un apport d'énergie de 12 à 28 Wh/kg, dans lequel on doit comprendre par le terme « masse sèche », un produit doté d'une humidité résiduelle inférieure à 5 % en poids, et la teneur en sulfate de la biomasse étant déterminée par l'intermédiaire de la teneur en soufre dans la biomasse et celle-ci étant déterminée à son tour selon la norme DIN EN ISO 11885.

2. Procédé selon la revendication 1, **caractérisé en ce que** l'extrusion est réalisée avec un apport d'énergie de 14 à 26, de préférence 16 à 24, en particulier 18 à 22 Wh/kg.

3. Procédé selon la revendication 1 ou 2, **caractérisé en ce que** la biomasse contenant des AGPI du taxon Labyrinthulomycetes en est une de la famille des Thraustochytriaceae, particulièrement préférablement des genres Thraustochytrium, Schizochytrium, Aurantiochytrium, Oblongichytrium ou Ulkenia, avant tout du genre Aurantiochytrium.

4. Procédé selon la revendication 3, **caractérisé en ce que** la biomasse contenant des AGPI en est une de l'espèce Aurantiochytrium limacinum, en particulier de la souche Aurantiochytrium limacinum SR21.

5. Procédé selon l'une quelconque des revendications précédentes, **caractérisé en ce que** la biomasse présente une teneur en sulfate, par rapport à la masse sèche, de 25 à 50 g/kg, préférablement 25 à 40, avant tout 25 à 35 g/kg.

6. Procédé selon l'une quelconque des revendications précédentes, **caractérisé en ce que** la composition utilisée lors de l'extrusion présente les caractéristiques suivantes :

a) une teneur totale en protéines de 33 à 67 % en poids, de préférence 39 à 61 % en poids, en particulier 44 à 55 % en poids ;
b) une teneur totale en graisses de 5 à 25 % en poids, de préférence 8 à 22 % en poids, en particulier 10 à 20 % en poids, avant tout 12 à 18 % en poids ;
c) une teneur totale en amidon de maximum 25 % en poids, en particulier de maximum 20 % en poids, de préférence de 6 à 17 % en poids, particulièrement préférablement de 8 à 14 % en poids ;
d) une teneur en biomasse de Labyrinthulomycetes, préférablement en biomasse de Thraustochytriaceae, de 2 à 24 % en poids, de préférence 4 à 22 % en poids, en particulier 9 à 20 % en poids, avant tout 11 à 18 % en poids ;
e) de préférence une teneur en acides gras polyinsaturés (AGPI) de 0,8 à 8 % en poids, de préférence 1,2 à 6 % en poids, en particulier 1,4 à 5 % en poids, avant tout 1,5 à 4 % en poids ;
f) de préférence une teneur en acides gras oméga 3 de 0,8 à 8 % en poids, de préférence 1,2 à 6 % en poids, en particulier 1,4 à 5 % en poids, avant tout 1,5 à 4 % en poids ;
g) de préférence une teneur en DHA de 0,1 à 4,0 % en poids, de préférence 0,25 à 3,0 % en poids, en particulier 0,5 à 2,8 % en poids, avant tout 0,8 à 2,5 % en poids, en particulier 1,0 à 2,0 % en poids.

7. Procédé selon l'une quelconque des revendications précédentes, **caractérisé en ce qu'**après l'extrusion et éventuellement le séchage de l'extrudat, un revêtement de l'extrudat est réalisé avec une huile, en particulier une huile végétale, de préférence en une quantité de 3 à 17 % en poids, en particulier 5 à 15 % en poids, par rapport au produit final.

8. Extrudat d'aliment pour animaux contenant des AGPI, qui comprend une biomasse contenant des AGPI du taxon Labyrinthulomycetes, **caractérisé en ce que** la biomasse présente une teneur en sulfate, par rapport à la masse sèche, de 25 à 60 g/kg et **en ce que** l'extrudat présente une capacité de charge d'huile d'au moins 0,25 g d'huile par g d'extrudat, de préférence d'au moins 0,30 g d'huile par g d'extrudat, particulièrement préférablement d'au moins 0,35 g d'huile par g d'extrudat, dans lequel on doit comprendre par le terme « masse sèche », un produit doté d'une humidité résiduelle inférieure à 5 % en poids, et la teneur en sulfate de la biomasse étant déterminée par l'intermédiaire de la teneur en soufre dans la biomasse et celle-ci étant déterminée à son tour selon la norme DIN EN ISO 11885.

9. Extrudat d'aliment pour animaux selon la revendication précédente, **caractérisé en ce que** la biomasse contenant des AGPI du taxon Labyrinthulomycetes est une biomasse de la famille des Thraustochytriaceae, particulièrement préférablement des genres Thraustochytrium, Schizochytrium, Aurantiochytrium, Oblongichytrium ou Ulkenia, avant tout du genre Aurantiochytrium.

10. Extrudat d'aliment pour animaux selon la revendication précédente, **caractérisé en ce que** la biomasse en est une de l'espèce Aurantiochytrium limacinum, en particulier de la souche Aurantiochytrium limacinum SR21.

11. Extrudat d'aliment pour animaux selon l'une quelconque des revendications précédentes, **caractérisé en ce que** la biomasse présente une teneur en sulfate, par rapport à la masse sèche, de 25 à 50 g/kg, préférablement 25 à 40, avant tout 25 à 35 g/kg.

**12.** Extrudat d'aliment pour animaux contenant des AGPI selon l'une quelconque des revendications précédentes, **caractérisé en ce qu'**il présente les caractéristiques suivantes :

a) une teneur totale en protéines de 30 à 60 % en poids, de préférence 35 à 55 % en poids, en particulier 40 à 50 % en poids ;
b) une teneur totale en graisses de 15 à 35 % en poids, de préférence 18 à 32 % en poids, en particulier 20 à 30 % en poids, avant tout 22 à 28 % en poids ;
c) une teneur totale en amidon de maximum 25 % en poids, en particulier de maximum 20 % en poids, de préférence de 5 à 15 % en poids, particulièrement préférablement de 7 à 13 % en poids ;
d) une teneur en biomasse de Labyrinthulomycetes, préférablement en biomasse de Thraustochytriaceae, de 2 à 22 % en poids, de préférence 4 à 20 % en poids, en particulier 8 à 18 % en poids, avant tout 10 à 16 % en poids ;
e) de préférence une teneur en acides gras polyinsaturés (AGPI) de 2 à 12 % en poids, de préférence 3 à 10 % en poids, en particulier 4 à 9 % en poids, avant tout 5 à 8 % en poids ;
f) de préférence une teneur en acides gras oméga 3 de 1 à 6 % en poids, de préférence 1,5 à 5 % en poids, en particulier 2 à 4,5 % en poids, avant tout 2,5 à 4 % en poids ;
g) de préférence une teneur en DHA de 0,5 à 3 % en poids, de préférence 0,8 à 2,5 % en poids, en particulier 1 à 2,5 % en poids, avant tout 1,2 à 2,2 % en poids, en particulier 1,2 à 2,0 % en poids.

**13.** Procédé pour les élevages d'animaux, en particulier de poissons, dans lequel un extrudat d'aliment pour animaux selon l'une quelconque des revendications précédentes est utilisé.

**IN DER BESCHREIBUNG AUFGEFÜHRTE DOKUMENTE**

**In der Beschreibung aufgeführte Patentdokumente**

- WO 2014122092 A

- WO 9408467 A

**In der Beschreibung aufgeführte Nicht-Patentliteratur**

- **BAEVERFJORD et al.** *Aquaculture,* 2006, vol. 261, 1335-1345
- *Klein: Seifen, Öle, Fette, Wachse,* 1968, vol. 94, 12

- **VON R.H. MÜLLER ; R. SCHUHMANN.** Teilchengrößenmessung in der Laborpraxis. *Wissenschaftliche Verlagsgesellschaft Stuttgart,* 1996
- **LEHRBUCH ; VON M. RHODES.** Introduction to Particle Technology. Verlag Wiley & Sons, 1998